Europäisches Patentamt

European Patent Office

Office européen des brevets

(11) Veröffentlichungsnummer : **0 198 344**
**B1**

(12) **EUROPÄISCHE PATENTSCHRIFT**

(45) Veröffentlichungstag der Patentschrift :
28.09.88

(51) Int. Cl.⁴ : **C 07 C 69/63, C 07 C 67/62, C 11 C 3/00**

(21) Anmeldenummer : **86104547.4**

(22) Anmeldetag : **03.04.86**

(54) **Stabilisierte Jodfette und Verfahren zu deren Herstellung.**

(30) Priorität : **13.04.85 DE 3513323**

(43) Veröffentlichungstag der Anmeldung :
**22.10.86 Patentblatt 86/43**

(45) Bekanntmachung des Hinweises auf die Patenterteilung : **28.09.88 Patentblatt 88/39**

(84) Benannte Vertragsstaaten :
**DE FR GB**

(56) Entgegenhaltungen :
**DE-B- 1 518 929**
**DE-C-   469 233**

(73) Patentinhaber : **Merck Patent Gesellschaft mit beschränkter Haftung**
**Frankfurter Strasse 250**
**D-6100 Darmstadt (DE)**

(72) Erfinder : **Kuhn, Walter, Dr.**
**Weidenweg 8**
**D-8750 Aschaffenburg (DE)**
Erfinder : **Härtner, Hartmut, Dr.**
**Mathildenweg 9**
**D-6109 Mühltal (DE)**
Erfinder : **Schindler, Fritz, Dr.**
**Am Kandelborn 1**
**D-8107 Reinheim 1 (DE)**
Erfinder : **Sandner, Ingo**
**Walbeweg 3**
**D-6100 Darmstadt (DE)**
Erfinder : **Hering, Klaus**
**Elisabethenstrasse 30**
**D-6100 Darmstadt (DE)**

## Beschreibung

Die Erfindung betrifft stabilisierte Iodfette und ein Verfahren zu ihrer Herstellung.

Iodierte Fette und Fettsäuren finden therapeutische Anwendung bei der Bekämpfung entzündlicher und rheumatischer Erkrankungen. Weiterhin werden sie diagnostisch als Kontrastmittel in der Radiographie eingesetzt.

Es ist eine Reihe von Verfahren zur Herstellung derartiger Iodfette bekannt. Die deutsche Patentschrift 96 495 lehrt die Darstellung von Iod- und Bromfetten durch Einwirkung von Iod- oder Brommonochlorid auf Fette. Aus der DPS 135 835 ist ein Iodierungsverfahren zu entnehmen, bei dem Fette mit gasförmigem Halogenwasserstoff zu Reaktion gebracht wird. Die direkte Umsetzung der freien Halogene unter Zusatz von schwefliger Säure wird in DPS 159 748 und unter Zusatz von Quecksilberoxid in DPS 202 790 beschrieben. Eine Kombination von Iod und Iodsäure als Iodierungsmittel ist in der DPS 199 549 offenbart.

Alle bisher bekannten Verfahren weisen eine Anzahl von Nachteilen auf. Mit Interhalogenverbindungen werden gemischthalogenhaltige Fette erhalten, deren Eigenschaften im Vergleich zu reinen Iodfetten in unerwünschter Weise differieren. Die Umsetzung von elementarem Iod in Gemischen mit Iodsäure, schwefliger Säure oder dem ökologisch bedenklichen Quecksilberoxid verlaufen nur unvollständig und führen zur Bildung unerwünschter Nebenprodukte. Ein Einsatz käuflichen gasförmigen Iodwasserstoffs scheitert an dessen hohem Preis. Bei der Eigendarstellung aus Iod, rotem Phosphor und Wasser entstehen als unerwünschte Nebenprodukte giftiger, selbstentzündlicher Phosphorwasserstoff sowie Phosphoniumiodid, das zu sublimieren und dadurch Öffnungen der Reaktionsapparatur zu verstopfen vermag.

Die Mehrzahl der nach den voranstehenden Verfahren hergestellten Iodfette und Iodfettsäuren sind darüberhinaus unzureichend stabil, d. h. sie unterliegen — auch unter Luft- und Lichtausschluß — bei der Lagerung innerhalb kurzer Zeit der Zersetzung, wodurch ihre therapeutische und diagnostische Verwendung stark beeinträchtigt wird.

Es besteht somit ein Bedürfnis nach einem Verfahren, welches die Herstellung von lagerstabilen Iodfetten in hohen Ausbeuten ohne Nebenproduktbildung und unter Verwendung wohlfeiler Hilfsstoffe erlaubt.

Diese Aufgabe wird gelöst durch das erfindungsgemäße Verfahren zur Herstellung von Iodfetten.

Gegenstand der Erfindung ist daher ein Verfahren zur Iodierung von Fetten, Fettsäuren oder deren Derivaten mittels Iodwasserstoff, dadurch gekennzeichnet, daß man die Iodierung mit wäßriger Iodwasserstoffsäure in Gegenwart eines wasserentziehenden Mittels durchführt und das erhaltene Iodierungsprodukt mit einem zur Stabilisierung ausreichenden Anteil an Ausgangsprodukt versetzt.

Die Durchführung der Reaktion gestaltet sich einfach. Als zu iodierende Ausgangsstoffe setzt man Fette pflanzlichen Ursprungs wie z. B. Kokosfett, Kakaobutter, Palmöl, Traubenkernöl, Erdnußöl, Leinöl, Mohnöl, Maiskernöl, Olivenöl, Rüböl, Sesamöl, Sonnenblumenöl und Weizenkeimöl oder tierischen Ursprungs wie z. B. Butterfett, Gänsefett, Rindertalg, Schmalz, Sardinenöl und Walöl ein. Geeignete Fettsäuren sind beispielsweise einfach oder mehrfach ungesättigte Fettsäuren wie Ölsäure, Erucasäure, Linolsäure, Linolensäure, Arachidonsäure oder Ricinolsäure. Für die Iodierung geeignete Fettsäurederivate stellen beispielsweise die Ester der vorgenannten Säuren mit ein- oder mehrwertigen Alkoholen dar. Besonders bevorzugte Ausgangsstoffe für die Iodierung nach dem erfindungsgemäßen Verfahren sind Mohnöl und Ölsäureethylester.

Die Konzentration der eingesetzten wässrigen Iodwasserstoffsäure ist nicht kritisch. Zweckmäßigerweise verwendet man höher konzentrierte Lösungen wie beispielsweise das 67 %ige Handelsprodukt.

Zum Wasserentzug sind alle unter den Verfahrensbedingungen gegenüber den Verfahrensausgangs- und -endprodukten stabilen Trockenmittel geeignet, insbesondere wasserärmere Derivate der Phosphorsäure wie Orthophosphorsäure, Polyphosphorsäure, Phosphorpentoxid oder auf ein Trägermaterial aufgebrachtes Phosphorpentoxid sowie Gemische dieser Stoffe. Hierfür gebräuchliche Trägermaterialien sind beispielsweise Kieselgel, Kieselgur, Aluminiumoxide und Zeolithe.

Überraschenderweise erleiden die Verfahrensausgangs- und -endprodukte durch die Anwesenheit stark phosphorsaurer Agenzien keinerlei Schaden wie z. B. Hydrolyse oder Bildung unerwünschter, die Stabilität und Reinheit der Endprodukte beeinträchtigender phosphorhaltiger Iodierungsprodukte.

Die Ausführung der Reaktion erfolgt durch Vereinigen des zu iodierenden Ausgangsproduktes mit der notwendigen Menge wässriger Iodwasserstoffsäure und Zugabe einer mindestens zur Bindung des vorhandenen Wassers ausreichenden Menge Trockenmittel zu dem gerührten Reaktionsgemisch. Ein etwaiger Überschuß an Iodwasserstoffsäure und/oder Trockenmittel ist nicht schädlich. Üblicherweise liegen die Reaktionstemperaturen bei 0° bis 50°, vorzugsweise bei 10 bis 30°.

Bei viskosen oder festen Ausgangsprodukten ist ein Zusatz eines inerten Lösungsmittels vorteilhaft. Zweckmäßigerweise trifft man die Auswahl aus den inerten, unter den gewählten Reaktionsbedingungen stabilen Lösungsmitteln, wie z. B. aliphatische oder aromatische Kohlenwasserstoffe, insbesondere Pentan, Hexan, Heptan, Cyclohexan, Benzol oder Toluol. Es können auch Gemische dieser Lösungsmittel zugesetzt werden.

Nach Durchführung der Reaktion trennt man zur Aufarbeitung die phosphorsäurehaltige Phase bzw. den das Trockenmittel enthaltenden Träger aus dem Reaktionsgemisch ab und wäscht die das Iodierungsprodukt enthaltende Phase mit Wasser säurefrei.

Zum Entfernen geringer Mengen gebildeten freien Iods kann erforderlichenfalls mit wässriger Natriumthiosulfatlösung entfärbt werden.

Das von etwa vorhandenem Lösungsmittel befreite Iodierungsprodukt wird schließlich mit einem zur Stabilisierung ausreichenden Anteil an Ausgangsprodukt versetzt. Die hierzu erforderliche Menge liegt vorzugsweise zwischen 0,5-5 Gew. %, insbesondere zwischen 1-3 Gew. %, bezogen auf die Menge Iodierungsprodukt. Sie kann jedoch erforderlichenfalls auch unter- oder überschritten werden. Unstabilisierte Iodierungsprodukte, die während des Lagerns eine Verfärbung erlitten haben, können durch Zusatz des Ausgangsproduktes wieder aufgehellt werden.

Derart stabilisierte Iodfette, Iodfettsäuren oder deren Derivate stellen einen weiteren Gegenstand der vorliegenden Erfindung dar.

In einer besonders bevorzugten Ausführungsform der Erfindung wird aus Oelsäureethylester mit wässriger Iodwasserstoffsäure und Polyphosphorsäure sowie durch Zusatz von Oelsäureethylester zum Iodierungsprodukt stabilisierter Iodoelsäureethylester hergestellt.

Mit der vorliegenden Erfindung steht somit ein sehr vorteilhaftes Verfahren zur einfachen und nebenproduktfreien Darstellung von stabilisierten Iodfetten, Iodfettsäureestern und deren Derivaten zur Verfügung.

Beispiel

Die Lösung von 310 g Oelsäureethylester in 500 ml Hexan wird mit 200 g 67 Gew. % HJ enthaltender Iodwasserstoffsäure unterschichtet und unter Rühren bei 20° portionsweise mit 250 g Polyphosphorsäure versetzt. Nach beendeter Zugabe rührt man noch 1 Stunde bei dieser Temperatur weiter.

Zur Aufarbeitung wird die Phosphorsäure als Unterphase abgetrennt und die organische Oberphase mit Wasser säurefrei gewaschen. Anschließendes Behandeln mit wässriger Natriumthiosulfatlösung entfärbt das Reaktionsprodukt vollständig. Mittels eines Dünnfilmverdampfers befreit man schonend vom Lösungsmittel und versetzt das so erhaltene Material mit 5 g Oelsäureester.

Auch nach mehrmonatiger Lagerung bei Tageslicht an der Luft sind keine Anzeichen einer Zersetzung oder Verfärbung zu erkennen. Eine nicht mit Ausgangsmaterial versetzte Probe Iodoelsäureester verfärbt sich nach ein bis zwei Tagen dunkelbraun bis schwarz.

Patentansprüche

1. Verfahren zur Iodierung von Fetten, Fettsäuren oder deren Derivaten mittels Iodwasserstoff, dadurch gekennzeichnet, daß man die Iodierung mit wässriger Iodwasserstoffsäure in Gegenwart eines wasserentziehenden Mittels durchführt und das erhaltene Iodierungsprodukt mit einem zur Stabilisierung ausreichenden Anteil an Ausgangsprodukt versetzt.

2. Verfahren nach Anspruch 1, dadurch gekennzeichnet, daß man als wasserentziehendes Mittel Polyphosphorsäure, Phosphorpentoxid oder auf ein Trägermaterial aufgebrachtes Phosphorpentoxid verwendet.

3. Verfahren nach einem der Ansprüche 1 und 2, dadurch gekennzeichnet, daß die Menge an zugesetztem uniodiertem Ausgangsprodukt 0,5-5 Gew.% bezogen auf die Menge Iodierungsprodukt beträgt.

4. Verfahren nach einem der Ansprüche 1 bis 3, dadurch gekennzeichnet, daß man als Fett Mohnöl einsetzt.

5. Verfahren nach einem der Ansprüche 1 bis 3, dadurch gekennzeichnet, daß man als Fettsäurederivat Oelsäureethylester einsetzt.

6. Stabilisierte Iodfette, Iodfettsäuren oder deren Derivate, gekennzeichnet durch einen Gehalt an zugesetztem uniodiertem Fett, Fettsäure oder deren Derivate.

7. Stabilisierte Iodfette, Iodfettsäuren oder deren Derivate nach Anspruch 6, dadurch gekennzeichnet, daß der Gehalt an zugesetztem uniodiertem Fett, Fettsäure oder deren Derivate 0,5-5 Gew.% bezogen auf die Menge Iodierungsprodukt beträgt.

8. Stabilisierter Iodoelsäureester nach einem der Ansprüche 6 und 7, dadurch gekennzeichnet, daß er 0,5-5 Gew.% zugesetzten Oelsäureester enthält.

Claims

1. Process for the iodination of fats, fatty acids or their derivatives by means of hydrogen iodide, characterized in that the iodination is performed with aqueous hydrogen iodide in presence of a dehydrating agent and a portion of starting material sufficient for stabilization is added to the iodination product obtained.

2. Process according to Claim 1 characterized in that polyphosphoric acid, phosphorus pentoxide or phosphorus pentoxide applied to a supporting material is used as dehydrating agent.

3. Process according to one of Claims 1 or 2, characterized in that the amount of added non-iodinated starting material is 0.5 to 5 percent by weight, referred to the amount of iodinated product.

4. Process according to one of Claims 1 to 3, characterized in that poppy-seed oil is used as fat.

5. Process according to one of Claims 1 to 3, characterized in that ethyl oleate is used as fatty acid derivative.

6. Stabilized iodinated fats, iodinated fatty acids or their derivatives, characterized by a content of added non-iodinated fat, fatty acid or their derivatives.

7. Stabilized iodinated fats, iodinated fatty acids or their derivatives according to Claim 6,

characterized in that the content of added non-iodinated fat, fatty acid or their derivatives is 0.5 to 5 percent by weight, referred to the amount of iodinated product.

8. Stabilized iodinated oleic acid ester according to one of Claims 6 and 7, characterized in that it contains 0.5 to 5 percent by weight of added oleic acid ester.

## Revendications

1. Procédé pour ioder des matières grasses, des acides gras ou leurs dérivés à l'aide d'acide iodhydrique, caractérisé en ce que l'on procède à l'iodation à l'aide d'acide iodhydrique aqueux en présence d'un agent déshydratant et on ajoute au produit d'iodation obtenu une proportion du produit de départ suffisante pour le stabiliser.

2. Procédé selon la revendication 1, caractérisé en ce que l'on utilise en tant qu'agent déshydratant l'acide polyphosphorique, l'anhydride phosphorique ou l'anhydride phosphorique appliqué sur une matière de support.

3. Procédé selon l'une des revendications 1 et 2, caractérisé en ce que la quantité de produit de départ non iodé qu'on ajoute est de 0,5 à 5 % en poids par rapport à la quantité du produit d'iodation.

4. Procédé selon l'une des revendications 1 à 3, caractérisé en ce que la matière grasse mise en œuvre est l'huile de pavot.

5. Procédé selon l'une des revendications 1 à 3, caractérisé en ce que le dérivé d'acide gras mis en œuvre est l'oléate d'éthyle.

6. Matières grasses iodées, acides gras iodés ou leurs dérivés, stabilisés, caractérisés en ce qu'ils ont été additionnés de matière grasse non iodée, d'acide gras non iodé ou de leurs dérivés non iodés.

7. Matières grasses iodées, acides gras iodés ou leurs dérivés, stabilisés, selon la revendication 6, caractérisés en ce que la teneur en matière grasse non iodée, acide gras non iodé ou dérivé non iodé ajouté est de 0,5 à 5 % en poids par rapport à la quantité du produit d'iodation.

8. Ester oléique iodé stabilisé selon l'une des revendications 6 et 7, caractérisé en ce qu'il contient de 0,5 à 5 % d'ester oléique ajouté.